# EUROPEAN PATENT SPECIFICATION

(11) **EP 1 607 676 B1**
(45) Date of publication and mention of the grant of the patent: **02.01.2019**
(21) Application number: 05012720.8
(22) Date of filing: 14.06.2005
(51) Int. Cl.: F21V 7/00, G02B 6/00, F21V 29/00

(54) **LED lamp with light pipes**
LED-Leuchte mit Lichtleitern
DEL-Lampe à guide de lumière

(30) Priority: 16.06.2004 US 580287 P; 27.07.2004 US 899546
(43) Date of publication of application: 21.12.2005
(73) Proprietor: OSRAM GmbH, 80807 München (DE)
(72) Inventor: Coushaine, Charles M., Rindge, NH 03461 (US); Tucker, Michael, Henniker, NH 03242 (US); Tessnow, Thomas, Weare, NH 03281 (US)

(56) References cited:
- EP-A- 1 389 710
- US-A- 3 772 506
- US-A- 4 463 410
- US-A- 5 438 485
- US-A- 5 725 296

## Description

### TECHNICAL FIELD

0002 This invention relates to light sources and more particularly to light sources employing light emitting diodes (LED or LEDs) and more particularly to light sources useful in the automotive field such as for headlights, taillights, stoplights, fog lights, turn signals, etc. Still more particularly, it relates to such light sources packaged to achieve industry accepted interchangeability.

### BACKGROUND ART

0003 In the past, most automotive light sources have involved the use of incandescent bulbs. While working well and being inexpensive, these bulbs have a relatively short life and, of course, the thin filament employed was always subject to breakage due to vibration.

0004 Recently some of the uses, particularly the stoplight, have been replaced by LEDs. These solid-state light sources have incredible life times, in the area of 100,000 hours, and are not as subject to vibration failures. However, these LED sources have been hard-wired into their appropriate location, which increases the cost of installation. It would therefore be an advance in the art if an LED light source could be provided that had the ease of installation of the incandescent light sources. It would be a still further advance in the art if an LED light source could be provided that achieved an industry accepted interchangeable standard to replace the aforementioned incandescent bulb.

Document EP 1 389 710 A discloses an LED light source according to the preamble of claim 1.

### DISCLOSURE OF INVENTION

0005 It is, therefore, an object of the invention to obviate the disadvantages of the prior art.

0006 It is another object of the invention to enhance LED light sources.

0007 Yet another object of the invention is the provision of an LED light source having good heat dissipation

0008 These objects are accomplished, in one aspect of the invention, by the provision of an LED light source comprising a housing having a base with a hollow core projecting from the base, the hollow core being substantially cylindrical and being surrounded by a plurality of elongated hollow tubes. The hollow core and the base are arrayed about a longitudinal axis 19. A printed circuit board is positioned in the base and has a plurality of LEDs operatively fixed thereto, each of the plurality of LEDs being positioned with one of the elongated hollow tubes in a one-to-one relationship at one end of the hollow core. A heat sink is positioned in a heat-transferring relationship with the printed circuit board, and a first reflector is attached to another, opposite end of the hollow core.

0009 Elimination of a former metal post that was necessary to carry the heat away from LEDs that were mounted to emit light directly at a reflector from a remote position greatly simplifies construction and reduces cost. Further, mounting the LEDs on a printed circuit board that is in direct contact with a heat sink removes heat more effectively. Additionally, the heat sink is mounted outside of the reflector, again aiding in the removal of heat from the entire lamp.

### BRIEF DESCRIPTION OF THE DRAWINGS

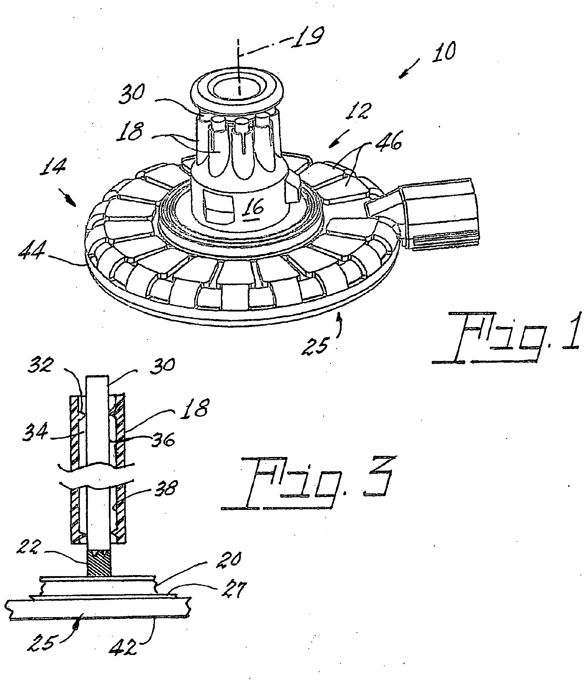

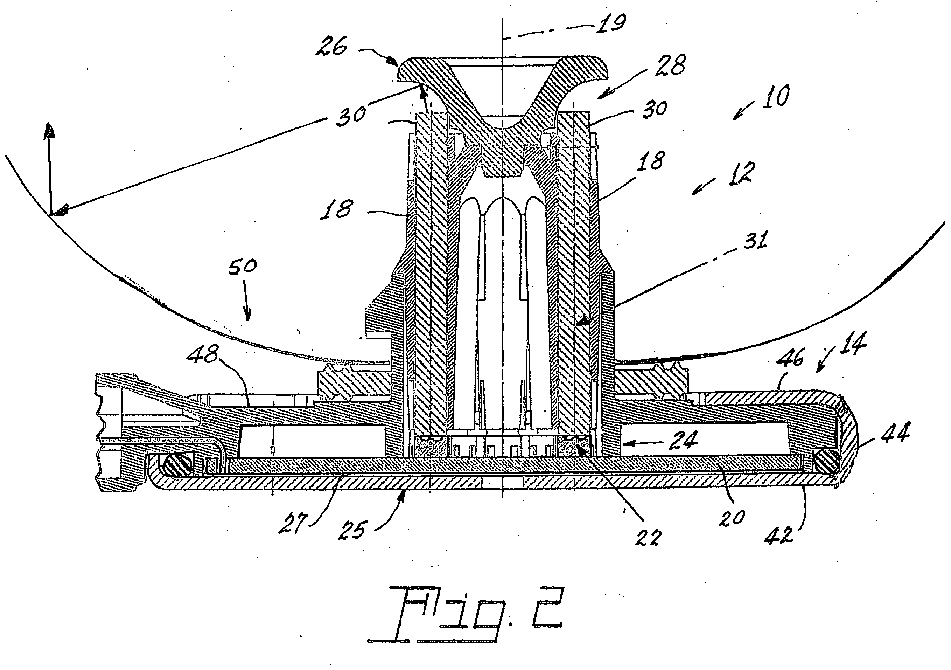

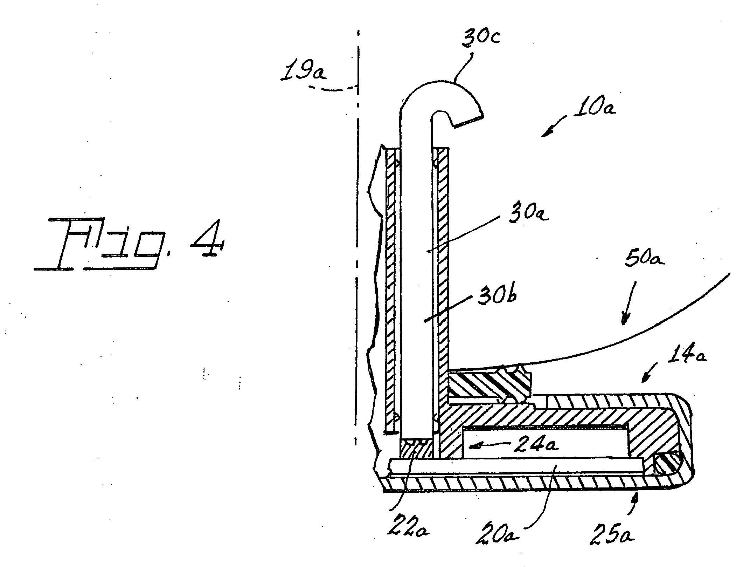

0010 Fig. 1 is a perspective view of an embodiment of the invention;
0011 Fig. 2 is an elevational sectional view of the embodiment of the invention of Fig. 1;
0012 Fig. 3 an enlarged sectional view of the light guides of the invention; and
0013 Fig. 4 is an elevational sectional view of an alternate embodiment of the invention.

### BEST MODE FOR CARRYING OUT THE INVENTION

0014 For a better understanding of the present invention, together with other and further objects, advantages and capabilities thereof, reference is made to the following disclosure and appended claims taken in conjunction with the above-described drawings.

0015 Referring now to the drawings with greater particularity, there is shown in Fig. 1 an LED light source 10 comprising a housing 12 having a base 14. A hollow core 16 projects from the base 14 and is substantially cylindrical and is surrounded by a plurality of elongated hollow tubes 18. The hollow core 16 and the base 14 are arrayed about a longitudinal axis 19. In a preferred embodiment of the invention there are eight tubes 18; however, the actual number of tubes will be dependent upon the light output of the individual LEDs and, as this light output increases, the number of tubes can be reduced. A printed circuit board 20 (see Fig. 2) is positioned in the base 14 and has a plurality of LEDs 22 operatively fixed thereto. Each of the plurality of LEDs 22 is positioned with one of the elongated hollow tubes 18 in a one-to-one relationship at one end 24 of the hollow core 16. A heat sink 25 is positioned in a heat-transferring relationship with the printed circuit board and a first reflector 26 is attached to another end 28 of the hollow core 16.

0016 To direct the light emitted by the LEDs from the source to the reflector 26, the interior of the hollow tubes 18 can be plated with a highly reflective material. However, in a preferred embodiment of the invention each of the hollow tubes 18 is fitted with a light guide 30, each light guide 30 extending from a position in intimate contact with one of the plurality of LEDs 22 to a position adjacent the reflector 26 along a longitudinal axis 31. All surfaces of the light guides are to be a polished finish so as to transmit the maximum amount of light from the LEDs to the reflector. As noted above, and as shown more clearly in Fig. 3, the light guides are in intimate contact with the LEDs.

0017 The light guides 30 can be any appropriate transparent material such as glass or plastic.

0018 Also, the reflector 26 can have its surface "A" changed to greatly alter the radiated light's appearance, thus providing great flexibility to the basic bulb.

0019 To insure that the maximum amount of emitted light is channeled through the light guides 30 each of the hollow tubes 18 is provided with inner protrusions 32 at each end for engaging the light guides 30, whereby the light guides have an air gap 34 between their outer surface 36 and the inner surface 38 of the hollow tubes 18. These features are most clearly shown in Fig. 3.

0020 The heat sink 25 is attached to the base 14 and in thermal contact with the printed circuit board 20. Preferably thermal putty 27 such as Thermagon 304 is used to make good thermal contact between the board 20 and the heat sink 25. The heat sink 25 has a bottom 42 with an upstanding side wall 44 terminating in a plurality of fingers 46, the fingers 46 being formed to overlie an upper surface 48 of the base 14 and can be of the type shown in co-pending patent application S.N. 10/838,090, filed 05/03/2004 and assigned to the assignee of the present invention.

0021 A second reflector 50 is positioned at end 24 of the hollow core 16, and has a concave curved surface 52 directed at the first reflector 26. In a preferred embodiment of the invention the concave curved surface can be parabolic. In another embodiment of the invention the reflector 26 can be eliminated and the light emanating from the LEDs could e directed to a projector optic.

0022 An alternate embodiment of the invention is shown in Fig. 4 wherein an LED light source 10a contains a plurality of light guides 30a spaced about a longitudinal axis 19a, each of the light guides 30a having a straight portion 30b and a curved portion 30c, the curved portion extending away from the longitudinal axis 19a;

0023 As with the previous embodiment, a printed circuit board 20a is positioned in a base 14a and has a plurality of LEDs 22a operatively fixed thereto, each of the plurality of LEDs 22 being positioned with one of the light guides 30a in a one-to-one relationship at one end 24a of the hollow core.

0024 A heat sink 25a positioned in a heat-transferring relationship with said printed circuit board and a reflector 50a is attached to the one end 24a of the hollow core 16a. Light that emanates from the curved portions 30c of the light guides 30a is directed toward the reflector 50a and then outward. The embodiment eliminates the reflector 26.

0025 While there have been shown and described what are present considered to be the preferred embodiments of the invention, it will be apparent to those skilled in the art that various changes and modifications can be made herein without departing from the scope of the invention as defined by the appended claims.

## Claims

1. An LED light source (10) comprising:
a housing (12) having a base (14);
a hollow core (16) projecting from said base (14), said hollow core (16) being substantially cylindrical **characterized in that**
said hollow core (16) being surrounded by a plurality of elongated hollow tubes (18),
said hollow core (16) and said base (14) being arrayed about a longitudinal axis (19);
a printed circuit board (20) positioned in said base (14) and having a plurality of LEDs (22) operatively fixed thereto, each of said plurality of LEDs (22) being positioned with one of said elongated hollow tubes (18) in a one-to-one relationship at one end (24) of said hollow core (16);
a heat sink (25) positioned in a heat-transferring relationship with said printed circuit board (20); and
a first reflector (26) attached to another end of said hollow core (16).

2. The LED light source of claim 1, wherein each of said hollow tubes (18) is fitted with a light guide (30), each light guide (30) extending from a position immediately above one of said plurality of LEDs (22) to a position adjacent said first reflector (26).

3. The LED light source of claim 2, wherein each of said hollow tubes (18) is provided with inner protrusions (32) at each end for engaging said light guides (30), whereby said light guides (30) have an air gap (34) between their outer surface (36) and the inner surface (38) of said hollow tubes (18).

4. The LED light source of claim 3, wherein a heat sink (25) is attached to said base (14) and in thermal contact with said printed circuit board (20), said heat sink (25) having a bottom (42) with an upstanding side wall (44) terminating in a plurality of fingers (46), said fingers (46) being formed to overlie an upper surface (48) of said base (14).

5. The LED light source of claim 4, wherein a second reflector (50) is positioned at said one end (24) of said hollow core (16), said second reflector (50) having a curved surface (52) directed at said first reflector (26).

6. The LED light source of claim 5, wherein said curved surface (52) is parabolic.

## Patentansprüche

1. LED-Lichtquelle (10), umfassend:
ein Gehäuse (12) mit einer Basis (14);
einen hohlen Kern (16), der aus der Basis (14) ragt, wobei der hohle Kern (16) im Wesentlichen zylindrisch ist;
**dadurch gekennzeichnet, dass**
der hohle Kern (16) von einer Vielzahl längserstreckender hohler Röhren (18) umgeben ist;
der hohle Kern (16) und die Basis (14) um eine Längsachse (19) gruppiert sind;
eine gedruckte Schaltung (20), die in der Basis (14) positioniert ist und eine Vielzahl von LEDs (22) aufweist, die daran funktional befestigt sind, wobei jede von der Vielzahl von LEDs (22) mit einer der längserstrecken hohlen Röhre (18) in einer 1:1-Beziehung an einem Ende (24) des hohlen Kerns (16) positioniert ist;
eine Wärmesenke (25), die in einer Wärmeübertragungsbeziehung mit der gedruckten Schaltung (20) positioniert ist; und
einen ersten Reflektor (26), der an einem anderen Ende des hohlen Kerns (16) befestigt ist.

2. LED-Lichtquelle nach Anspruch 1, wobei jede der hohlen Röhren (18) mit einem Lichtleiter (30) ausgestattet ist, wobei jeder Lichtleiter (30) sich von einer Position unmittelbar oberhalb von einer der Vielzahl von LEDs (22) zu einer Position erstreckt, die zu dem ersten Reflektor (26) benachbart ist.

3. LED-Lichtquelle nach Anspruch 2, wobei jede der hohlen Röhren (18) mit inneren Vorsprüngen (32) an jedem Ende ausgestattet ist, um in Eingriff mit den Lichtleitern (30) zu kommen, wobei die Lichtleiter (30) einen Luftspalt (34) zwischen ihrer Außenfläche (36) und der Innenfläche (38) der hohlen Röhren (18) aufweisen.

4. LED-Lichtquelle nach Anspruch 3, wobei eine Wärmesenke (25) an der Basis (14) und in thermischem Kontakt mit der gedruckten Schaltung (20) befestigt ist, wobei die Wärmesenke (25) einen Boden (42) mit einer aufrechten Seitenwand (44) aufweist, die in einer Vielzahl von Fingern (46) endet, wobei die Finger (46) so gebildet sind, dass sie über einer Oberseite (48) der Basis (14) liegen.

5. LED-Lichtquelle nach Anspruch 4, wobei ein zweiter Reflektor (50) an dem einen Ende (24) des hohlen Kerns (16) positioniert ist, wobei der zweite Reflektor (50) eine gekrümmte Oberfläche (52) aufweist, die auf den ersten Reflektor (26) gerichtet ist.

6. LED-Lichtquelle nach Anspruch 5, wobei die gekrümmte Oberfläche (52) parabolisch ist.

## Revendications

1. Une source de lumière à LED (10) comprenant :
un logement (12) possédant une base (14),
un noyau creux (16) en saillie à partir de ladite base (14), ledit noyau creux (16) étant sensiblement cylindrique,
**caractérisée en ce que**
ledit noyau creux (16) est entouré par une pluralité de tubes creux allongés (18),
ledit noyau creux (16) et ladite base (14) étant disposés en matrice autour d'un axe longitudinal (19),
une carte à circuits imprimés (20) positionnée dans ladite base (14) et possédant une pluralité de LED (22) fixées de manière opérationnelle à celle-ci, chaque LED de ladite pluralité de LED (22) étant positionnée avec un desdits tubes creux allongés (18) dans une relation un à un au niveau d'une extrémité (24) dudit noyau creux (16),
un dissipateur thermique (25) positionné dans une relation de transfert thermique avec ladite carte à circuits imprimés (20), et
un premier réflecteur (26) fixé à une autre extrémité dudit noyau creux (16).

2. La source de lumière à LED selon la revendication 1, dans laquelle chacun desdits tubes creux (18) est muni d'un guide de lumière (30), chaque guide de lumière (30) s'étendant à partir d'une position immédiatement au-dessus d'une LED de ladite pluralité de LED (22) vers une position adjacente audit premier réflecteur (26).

3. La source de lumière à LED selon la revendication 2, dans laquelle chacun desdits tubes creux (18) est muni de saillies internes (32) au niveau de chaque extrémité pour une mise en prise avec lesdits guides de lumière (30). grâce à quoi lesdits guides de lumière (30) possèdent un entrefer (34) entre leur surface extérieure (36) et la surface intérieure (38) desdits tubes creux (18).

4. La source de lumière à LED selon la revendication 3, dans laquelle un dissipateur thermique (25) est fixé à ladite base (14) et en contact thermique avec ladite carte à circuits imprimés (20), ledit dissipateur thermique (25) possédant une partie inférieure (42) avec une paroi latérale verticale (44) se terminant en une pluralité de doigts (46), lesdits doigts (46) étant formés de façon à recouvrir une surface supérieure (48) de ladite base (14).

5. La source de lumière à LED selon la revendication 4, dans laquelle un deuxième réflecteur (50) est positionné au niveau de ladite extrémité (24) dudit noyau creux (16), ledit deuxième réflecteur (50) possédant une surface incurvée (52) dirigée vers ledit premier réflecteur (26).

6. La source de lumière à LED selon la revendication 5, dans laquelle ladite surface incurvée (52) est parabolique.
